**Europäisches Patentamt**

**European Patent Office**

**Office européen des brevets**

(19)

(11) Veröffentlichungsnummer: **0 305 812 B1**

## EUROPÄISCHE PATENTSCHRIFT

(12)

(45) Veröffentlichungstag der Patentschrift: **18.03.92**

(51) Int. Cl.⁵: **C07C 205/44**, C07C 201/16

(21) Anmeldenummer: **88113378.9**

(22) Anmeldetag: **18.08.88**

(54) **Verfahren zur Reinigung von Nitrobenzaldehyd.**

(30) Priorität: **29.08.87 DE 3728926**

(43) Veröffentlichungstag der Anmeldung:
**08.03.89 Patentblatt 89/10**

(45) Bekanntmachung des Hinweises auf die
Patenterteilung:
**18.03.92 Patentblatt 92/12**

(84) Benannte Vertragsstaaten:
**CH DE FR GB LI SE**

(56) Entgegenhaltungen:

**J. ORG. CHEM., Band 21, 1956, Seiten
1445-1447; J.M. SUGIHARA et al.:
"Recrystallization of organic compounds
from detergent-water systems"**

**Chemiker-Kalender, S.158/159 u. S.308/309,
Springer-Verlag 1956**

(73) Patentinhaber: **BAYER AG**

**W-5090 Leverkusen 1 Bayerwerk(DE)**

(72) Erfinder: **Streicher, Willi, Dr.
Andreas-Gryphius-Strasse 7
W-5000 Köln 80(DE)**
Erfinder: **Blank, Heinz Ulrich, Dr.
Am Geusfelde 35
W-5068 Odenthal-Gloebusch(DE)**

EP 0 305 812 B1

**Beschreibung**

Die Erfindung betrifft ein Verfahren zur Reinigung von rohem Nitrobenzaldehyd mit einem Gehalt an den unerwünschten Stellungsisomeren durch Behandlung mit Wasser in Gegenwart eines Emulgators.

Aus Houben-Weyl, Methoden der organischen Chemie, Band X/1, S. 600 (1971) ist es bekannt, daß bei der Nitrierung von Benzaldehyd ein Gemisch der drei möglichen Isomeren entsteht.

Zur Reinigung des gewünschten Nitrobenzaldehyds kann eine Umkristallisation aus organischen Lösungsmitteln vorgenommen werden. Hierbei ergeben sich erhebliche Verluste an dem gewünschten Produkt und beträchtliche Lösungsmittelkosten. Beim Arbeiten in großtechnischem Maßstab sind zusätzliche Maßnahmen im Hinblick auf die Betriebssicherheit, die Rückgewinnung des Lösungsmittels, den Gesundheits- und den Umweltschutz erforderlich.

In J. Org. Chem. 21 (1956), S. 1445 - 1447 wird die Umkristallisation organischer Materialien aus Wasser in Gegenwart von Detergentien diskutiert. Es wurde beobachtet, daß bei organischen Verbindungen mit begrenzter Wasserlöslichkeit die Kristallisationsrate in Gegenwart von oberflächenaktiven Agentien stark abgesenkt wird. Die erforderliche Prozedur ist umständlich und gekennzeichnet dadurch, daß die organische Substanz auch beim Erhitzen zum Siedepunkt nur langsam in Lösung geht, daß jedoch eine vollständige Lösung vorliegen muß, was gegebenenfalls durch Heißfiltration ungelöster Anteile erreicht wird. Sollte im heißen Filtrat die Kristallisation beginnen, wird noch einmal zur vollständigen Auflösung erhitzt und langsam abgekühlt. Angaben über Art und Menge der ursprünglichen Verunreinigungen und Angaben über den erzielten Reinheitsgrad sind nicht angegeben.

Es wurde nun überraschend gefunden, daß es zur Reinigung von rohem Nitrobenzaldehyd von seinen unerwünschten Stellungsisomeren nicht erforderlich ist, das Ausgangsmaterial vollständig in Lösung zu bringen, daß also keine Umkristallisation erforderlich ist. Es ist vielmehr ausreichend, in einem Zweiphasensystem mit einem Anteil an nicht gelöstem Nitrobenzaldehyd zu arbeiten. Diese Arbeitsweise ergibt beträchtliche Vorteile, unter anderem in der Raum-Zeit-Ausbeute. Der Reinigungseffekt ist erheblich und ist für ein Zweiphasensystem mit unübersichtlichen Oberflächenvorgängen an der nicht gelösten Phase sehr überraschend.

Die Erfindung betrifft daher ein Verfahren zur Reinigung von rohem Nitrobenzaldehyd, der durch seine Stellungsisomeren verunreinigt ist, das dadurch gekennzeichnet ist, daß man den rohen Nitrobenzaldehyd mit 100 - 10 000 Gew-% Wasser und 1 -30 Gew.-% Emulgator, alles bezogen auf das Trockengewicht des rohen Nitrobenzaldehyds, bei einer Temperatur von 20 - 140°C und einem pH-Wert von 3 - 14 so behandelt, daß ein Zweiphasensystem mit einem Anteil an nicht gelöstem Nitrobenzaldehyd vorliegt.

Das erfindungsgemäße Verfahren wird bei einer Temperatur von 20 - 140°C, bevorzugt 40 - 120°C, drucklos oder unter Druck, kontinuierlich oder diskontinuierlich durchgeführt. Man verwendet 100 - 10 000 Gew-.%, bevorzugt 150 - 3000 Gew.-%, besonders bevorzugt 150 - 800 Gew.-% Wasser und 1 - 30 Gew.-%, bevorzugt 2 - 20 Gew.-% Emulgator, alles bezogen auf das Trockengewicht des rohen Nitrobenzaldehyds. Erfindungsgemäß wird ein pH-Bereich von 3 - 14, bevorzugt 5 - 13, eingestellt.

Emulgatoren für das erfindungsgemäße Verfahren sind beispielsweise solche, wie sie in Ullmanns Enzyklopädie der Technischen Chemie, 4. Auflage, Band 10 (1975), S. 455 bis 464 beschrieben sind. Hierzu zählen kationische Emulgatoren, anionische Emulgatoren und nicht-ionische Emulgatoren. Kationische Emulgatoren sind beispielsweise primäre, sekundäre oder tertiäre Fettaminsalze, Salze von Alkylendiaminen und von Polyaminen, quartäre Alkylammoniumsalze, Alkylbenzylammoniumsalze, quartäre Amidoaminverbindungen, Alkanolaminsalze von Ethern und Estern, Alkylpyridiniumsalze, u.a.. Anionische Emulgatoren sind beispielsweise Carboxylate, wie Seifen, Salze fluorierter Carbonsäuren, Alkoxycarboxylate, Sulfoseifen, Sulfonamidocarbonsäuren; Sulfonate, wie Fettsäuresulfonate, Fettsäureestersulfonate, Salze von Sulfobernsteinsäureestern, perfluorierte Alkylsulfonate, Alkylbenzolsulfonate; Sulfate, wie sulfatierte Seifen, Ester, Fettsäureamide, primäre und sekundäre Alkohole und Alkanolamide; Phosphorsäure-Verbindungen wie Phosphate von Alkoholen, Phenolen oder deren Polyglykolethern, Phosphorsäureester von Glykolen und Glyceriden, Ester der phosphorigen Säure u.a.. Nicht-ionische Emulgatoren sind beispielsweise Fettsäureester von Alkoholen, Ethylenglykol, Polyethylenglykol, Propylenglykol, Glycerin; Fettamine und Fettsäureamide, einschließlich von Fettsäurealkanolamiden und Polyaminen, Polyglykolether von Alkoholen, Thioalkoholen, Fettsäuren, Fettsäureestern, Fettsäureaminen, Fettsäurealkanolamiden, Fetten und Ölen, Polypropylenglykol, kondensierten Phenolen, kondensierten Aminen u.a. sowie die entsprechenden Polypropylenglykolether der genannten Verbindungsklassen.

Das erfindungsgemäße Verfahren ist gleichermaßen auf rohen o-, m- oder p-Nitrobenzaldehyd anwendbar. Die rohen Nitrobenzaldehyde enthalten 0,1 - 15 Gew.-%, bevorzugt 0,1 - 10 Gew.-%, besonders bevorzugt 0,1 - 8 Gew.-% der jeweiligen Stellungsisomere, bezogen auf das Trockengewicht des rohen Nitrobenzaldehyds, als Verunreinigung. Als unerwünschte Stellungsisomere gelten beispielsweise bei o-Nitrobenzaldehyd der m- und der p-Nitrobenzaldehyd. Von den unerwünschten Stellungsisomeren kann entweder nur eines vorhanden sein oder es liegen beide vor. Der letztere Fall dürfte die Regel sein.

Selbstverständlich kann auch wasserfeuchter roher Nitrobenzaldehyd im erfindungsgemäßen Verfahren eingesetzt werden. Der Wassergehalt des trockenen oder wasserfeuchten rohen Nitrobenzaldehyds beträgt 0 - 1000 Gew.-%, beispielsweise 0,1 - 1000 Gew.-%, bevorzugt 1 - 800 Gew.-%, besonders bevorzugt 5 - 600 Gew.-% Wasser, bezogen auf das Trockengewicht des rohen Nitrobenzaldehyds. Solche Wassergehalte werden auf die Menge des für die erfindungsgemäße Behandlung erforderlichen Wassers angerechnet. Geringe Mengen an Säuren, etwa von der Nitrierungsstufe des rohen Nitrobenzaldehyds, oder an Alkali, etwa als Überschuß aus einer Neutralisation solcher Säuren, sowie an Salzen, etwa aus einer solchen Neutralisation, sind für das erfindungsgemäße Verfahren unschädlich. Sollte hierdurch der pH-Wert außerhalb des erfindungsgemäßen Bereiches liegen, wird er durch zumindest partielle Neutralisation in den erfindungsgemäßen Bereich gebracht.

Selbstverständlich kann der rohe Nitrobenzaldehyd auch in Form der Acetale eingesetzt werden, die zweckmäßig vorab hydrolysiert werden.

Das erfindungsgemäße Verfahren kann beispielsweise wie folgt durchgeführt werden: Der rohe Nitrobenzaldehyd wird mit der erforderlichen Menge Wasser und Emulgator versetzt, wobei etwa eingebrachtes Wasser angerechnet wird. Man kann jedoch auch zuerst den Emulgator und dann das Wasser zum Nitrobenzaldehyd geben. Weiterhin ist es auch möglich, den Nitrobenzaldehyd, das Wasser und den Emulgator simultan zu dosieren. Die Dosierung der genannten Komponenten erfolgt beispielsweise bei 10 - 140°C, bevorzugt bei 15 - 120°C. Die gewünschte Reaktionstemperatur und der gewünschte pH-Wert werden eingestellt. Nach dem Abkühlen wird der als feste Phase vorliegende gereinigte Nitrobenzaldehyd in üblicher Weise, beispielsweise durch Filtration, von der wäßrigen Phase abgetrennt, gewaschen oder nach erneuter Aufschlämmung in Wasser erneut filtriert und schließlich getrocknet.

Die im rohen Nitrobenzaldehyd als Verunreinigung enthaltenen Stellungsisomeren gehen in ihrer Menge auf 80 % ihres ursprünglichen wertes zurück, vielfach auf 60 % oder noch weniger. Bei hohen Verunreinigungen wird im allgemeinen eine Emulgatormenge im oberen Teil des oben beschriebenen Bereiches angewandt; für den Fall geringerer Reinheitsanforderungen kann jedoch auch weniger Emulgator eingesetzt werden.

Nitrobenzaldehyde sind dem Fachmann als wertvolle Ausgangsstoffe beispielsweise für die Herstellung von Farbstoffen wohlbekannt.

Beispiele

Beispiel 1

190,0 g wasserfeuchter m-Nitrobenzaldehyd (enthaltend 163,0 g rohen Nitrobenzaldehyd der Zusammensetzung: 1,5 Gew.-% o-Isomeres; 2,3 Gew.-% p-Isomeres; 96,2 Gew.-% m-Isomeres) wurden in 445 g Wasser suspendiert und mit 20,0 g eines Emulgators, der durch Kondensation von m-Kresol, Formaldehyd, Natriumsulfit und 2-Naphthol-6-sulfonsäure hergestellt wurde, versetzt. Die Suspension wurde auf 60°C erhitzt und mit NaOH auf pH 10,4 gestellt. Anschließend wurde zum Rückfluß erhitzt und 30 Min. unter Rückfluß gerührt. Danach wurde auf 40°C abgekühlt, 2,5 Std. bei 40°C gerührt und abgesaugt. Das Filtergut wurde mit Wasser gewaschen und erneut abgesaugt. Nach der Trocknung wurden 141,7 g m-Nitrobenzaldehyd erhalten.

Gehalt in Gew.-%

0,06 o-Nitrobenzaldehyd
99,8 m-Nitrobenzaldehyd
0,1 p-Nitrobenzaldehyd
Der Rest zu 100 % besteht aus nicht näher untersuchten Komponenten (gilt auch für die weiteren Beispiele).
Ausbeute: 93,1 % (bezogen auf eingesetzten m-Nitrobenzaldehyd)

Beispiel 2

190,0 g wasserfeuchter m-Nitrobenzaldehyd der Zusammensetzung wie in Beispiel 1 wurden in 445,0 g Wasser suspendiert und mit 10,0 g des in Beispiel 1 verwendeten Emulgators versetzt und entsprechend der in Beispiel 1 beschriebenen Verfahrensweise gereinigt.

Es wurden nach Trocknung 144,6 g m-Nitrobenzaldehyd erhalten.

Gehalt in Gew.-%

0,08 o-Nitrobenzaldehyd
99,7 m-Nitrobenzaldehyd
0,2 p-Nitrobenzaldehyd
Ausbeute: 95 % (bezogen auf eingesetzten m-Nitrobenzaldehyd)

Beispiel 3 (zum Vergleich)

190,0 g wasserfeuchter m-Nitrobenzaldehyd der Zusammensetzung wie in Beispiel 1 wurden in 445,0 g Wasser suspendiert und ohne Emulgator entsprechend der in Beispiel 1 beschriebenen Verfahrensweise gereinigt.
Nach Trocknung wurden 147,4 g m-Nitrobenzaldehyd erhalten.

Gehalt in Gew.-%

0,8 o-Nitrobenzaldehyd
97,6 m-Nitrobenzaldehyd
1,6 p-Nitrobenzaldehyd
Ausbeute: 95,3 % (bezogen auf eingesetzten m-Nitrobenzaldehyd)

Beispiel 4

Zu 211,0 g m-Nitrobenzaldehyd-dimethylacetal der Zusammensetzung: 1,6 Gew.-% o-Isomeres; 2,4 Gew.-% p-Isomeres; 95,6 Gew.-% m-Isomeres; 0,4 Gew.-% sonstige Verunreinigungen wurden 63,4 g Wasser und 1,63 g Monohydrat (100 %ige $H_2SO_4$) dosiert. Danach wurde unter Rühren erhitzt und das aus der Acetalspaltung entstandene Methanol-Wasser-Gemisch bei einer Sumpftemperatur von 80 -106 °C über eine einfache Destillationseinrichtung abdestilliert.
Nach der Destillation wurden zum Destillationsrückstand 445,0 g Wasser gegeben; es wurde mit Natronlauge neutralisiert und es wurden 10,0 g des in Beispiel 1 verwendeten Emulgators zudosiert; dann wurde 30 Min. unter Rückfluß gerührt. Die Emulsion wurde unter Rühren auf 40 °C abgekühlt und 2,5 Std. bei 40 °C gerührt. Der ausgefallene m-Nitrobenzaldehyd wurde abgesaugt, mit Wasser gewaschen und erneut abgesaugt.
Nach der Trocknung wurden 144,3 g m-Nitrobenzaldehyd erhalten.

Gehalt in Gew.-%

0,05 o-Nitrobenzaldehyd
99,6 m-Nitrobenzaldehyd
0,3 p-Nitrobenzaldehyd
Ausbeute: 92,5 % (bezogen auf eingesetzten m-Nitrobenzaldehyd)

Beispiel 5

Zu 211,0 g m-Nitrobenzaldehyd-dimethylacetal der Zusammensetzung wie in Beispiel 4 wurden 63,4 g Wasser und 1,63 g Monohydrat (100 %ige $H_2SO_4$) dosiert. Danach wurde unter Rühren erhitzt und das aus der Acetalspaltung entstandene Methanol-Wasser-Gemisch bei einer Sumpftemperatur von 80-106 °C über eine einfache Destillationseinrichtung abdestilliert. Nach der Destillation wurden zum Destillationsrückstand 445,0 g Wasser gegeben; es wurde mit Natronlauge neutralisiert und es wurden 10,0 g Disek.-butyl-napthalin-sulfonsäure-Natriumsalz zugegeben. Dann wurde weiter wie in Beispiel 4 verfahren.
Nach der Trocknung wurden 145,7 g m-Nitrobenzaldehyd erhalten.

Gehalt in Gew.-%

0,2 o-Nitrobenzaldehyd
98,9 m-Nitrobenzaldehyd

0,4 p-Nitrobenzaldehyd
Ausbeute: 93,2 % (bezogen auf eingesetzten m-Nitrobenzaldehyd)

Beispiel 6

211,0 g m-Nitrobenzaldehyd-dimethylacetal der Zusammensetzung wie in Beispiel 4 wurden entsprechend Beispiel 4 umgesetzt. Als Emulgator wurden 10,0 g Benzyl-dodecyldimethyl-ammoniumchlorid verwendet.
Nach der Trocknung wurden 145,2 g m-Nitrobenzaldehyd erhalten.

Gehalt in Gew.-%

0,1 o-Nitrobenzaldehyd
99,1 m-Nitrobenzaldehyd
0,7 p-Nitrobenzaldehyd
Ausbeute: 93,1 % (bezogen auf eingesetzten m-Nitrobenzaldehyd)

Beispiel 7

Zu 211,0 g m-Nitrobenzaldehyd-dimethylacetal der Zusammensetzung: 1,3 Gew.-% o-Isomeres; 5,6 Gew.-% p-Isomeres; 92,7 Gew.-% m-Isomeres; 0,4 Gew.-% sonstige Verunreinigungen wurden 63,4 g Wasser und 1,63 g Monohydrat (100 %ige $H_2SO_4$) dosiert. Danach wurde unter Rückfluß erhitzt und das aus der Acetalspaltung entstandene Methanol-Wasser-Gemisch bei einer Sumpftemperatur von 80 - 106°C über eine einfache Destillationseinrichtung abdestilliert. Nach der Destillation wurden zum Destillationsrückstand 445,0 g Wasser gegeben; es wurde mit Natronlauge neutralisiert, und 20,0 g des in Beispiel 1 verwendeten Emulgators wurden zudosiert. Die Emulsion wurde 30 Min. bei Rückfluß gerührt, unter Rühren auf 40°C abgekühlt und 2,5 Std. bei 40°C gerührt. Der ausgefallene m-Nitrobenzaldehyd wurde abgesaugt, mit Wasser gewaschen und erneut abgesaugt.
Nach der Trocknung erhielt man 133,7 g m-Nitrobenzaldehyd.

Gehalt in Gew.-%

99,4 m-Nitrobenzaldehyd
0,4 p-Nitrobenzaldehyd
Rest sonstige Verunreinigungen
Ausbeute: 88,6 % (bezogen auf eingesetzten m-Nitrobenzaldehyd)

Beispiele 8 - 10

Nach der grundsätzlichen Arbeitsweise von Beispiel 1 wurden jeweils 75,5 g 90 %iger m-Nitrobenzaldehyd mit 5 % o-Isomerem und 5 % p-Isomerem und der in Beispiel 1 genannte Emulgator eingesetzt. Die Tabelle 1 nennt die Mengen an Wasser und Emulgator, die Zusammensetzung des Produktes und die Ausbeute in % der theoretischen Ausbeute.

Tabelle 1

| Reinigung von m-Nitrobenzaldehyd | | | | | | | |
|---|---|---|---|---|---|---|---|
| Beispiel | $H_2O$ | Emulgator | | Produkt (Gew.-%) | | | Ausbeute |
| | (Gew.-%) | (g) | (Gew.-%) | o- | m- | p- | (%) |
| 8 | 200 | 15,1 | 20 | 1,5 | 97,0 | 1,5 | 82,0 |
| 9 | 700 | 15,1 | 20 | 1,3 | 97,5 | 1,2 | 82,0 |
| 10 | 700 | 2,3 | 3 | 2,2 | 94,8 | 3,0 | 90,0 |

Beispiele 11 - 21

In gleicher Weise wie in den Beispielen 8 - 10 wurden jeweils 75,5 g o-Nitrobenzaldehyd der angegebenen Zusammensetzung und der in Beispiel 1 genannte Emulgator eingesetzt. Die Tabelle 2 enthält alle weiteren Angaben.

Beispiele 22 - 25

In gleicher Weise wie in den Beispielen 8 - 10 wurden jeweils 75,5 g p-Nitrobenzaldehyd der angegebenen Zusammensetzung und der in Beispiel 1 genannte Emulgator eingesetzt. Die Tabelle 3 enthält alle weiteren Angaben.

Tabelle 2

| Reinigung von o-nitrobenzaldehyd | | | | | | | | | | |
|---|---|---|---|---|---|---|---|---|---|---|
| Beispiel | Einsatz (Gew.-%) | | | $H_2O$ | Emulgator | | Produkt (Gew.-%) | | | Ausbeute |
| | o- | m- | p- | (Gew.-%) | (g) | (Gew.-%) | o- | m- | p- | (%) |
| 11 | 96 | 3 | 1 | 273 | 5,0 | 6,3 | 99,1 | 0,8 | 0,1 | 95 |
| 12 | 96 | 3 | 1 | 273 | 10,0 | 12,6 | 99,7 | 0,3 | - | 92 |
| 13 | 90 | 5 | 5 | 200 | 15,1 | 20,0 | 95,5 | 2,2 | 2,3 | 87 |
| 14 | 90 | 5 | 5 | 700 | 15,1 | 20,0 | 95,9 | 1,4 | 2,7 | 87 |
| 15 | 90 | 5 | 5 | 700 | 2,3 | 3,0 | 92,5 | 3,7 | 3,8 | 95,3 |
| 16 | 90 | 5 | 5 | 700 | 30,2 | 40,0 | 98,5 | 0,8 | 0,7 | 80,4 |
| 17 | 96 | 3 | 1 | 273 | - | - * | 96,2 | 2,9 | 0,9 | 98 |
| 18 | 96 | 3 | 1 | 273 | 5,0 | 6,3** | 98,6 | 1,2 | 0,2 | 97,5 |
| 19 | 96 | 3 | 1 | 273 | 5,0 | 6,3*** | 98,4 | 1,3 | 0,3 | 97 |
| 20 | 95 | 2,5 | 2,5 | 700 | 10,0 | 12,6 | 98,7 | 0,7 | 0,6 | 91 |
| 21 | 95 | 2,5 | 2,5 | 700 | 15,1 | 20,0 | 99,4 | 0,4 | 0,2 | 89,5 |

* ohne Emulgator; Vergleichsversuch

** Emulgator Zephirol: Benzyl-dodecyl-dimethyl-ammoniumchlorid

*** Emulgator Erkantol BXG: Di-sek.-butyl-naphthalin-sulfonsäure-Natriumsalz

Tabelle 3

| Reinigung von p-Nitrobenzaldehyd | | | | | | | | | | |
|---|---|---|---|---|---|---|---|---|---|---|
| Beispiel | Einsatz (Gew.-%) | | | $H_2O$ | Emulgator | | Produkt (Gew.-%) | | | Ausbeute |
| | o- | m- | p- | (Gew.-%) | (g) | (Gew.-%) | o- | m- | p- | (%) |
| 22 | 5 | 5 | 90 | 700 | 5,0 | 6,3 | 1,3 | 2,0 | 96,7 | 96 |
| 23 | 5 | 5 | 90 | 700 | 15,1 | 20 | 0,6 | 1,0 | 98,4 | 95 |
| 24 | 5 | 5 | 90 | 700 | 22,7 | 30 | 0,3 | 1,3 | 98,4 | 92,7 |
| 25 | 2,5 | 2,5 | 95 | 700 | 15,1 | 20 | - | 0,3 | 99,7 | 95 |

**Patentansprüche**

1.  Verfahren zur Reinigung von rohem Nitrobenzaldehyd, der durch seine Stellungsisomeren verunreinigt ist, dadurch gekennzeichnet, daß man den rohen Nitrobenzaldehyd mit 100 - 10 000 Gew.-% Wasser und 1 - 30 Gew.-% Emulgator, alles bezogen auf das Trockengewicht des rohen Nitrobenzaldehyds, bei einer Temperatur von 20 -140°C und einem pH- Wert von 3 - 14 so behandelt, daß ein Zweiphasensystem mit einem Anteil an nicht gelöstem Nitrobenzaldehyd vorliegt.

2.  Verfahren nach Anspruch 1, dadurch gekennzeichnet, daß der rohe Nitrobenzaldehyd 0,1 - 15 Gew.-% unerwünschte Stellungsisomere enthält.

6

3. Verfahren nach Ansprüchen 1 und 2, dadurch gekennzeichnet, daß der rohe Nitrobenzaldehyd 0,1 - 10 Gew.-% unerwünschte Stellungsisomere enthält.

4. Verfahren nach Ansprüchen 1 bis 3, dadurch gekennzeichnet, daß der rohe Nitrobenzaldehyd 0,1 - 8 Gew.-% unerwünschte Stellungsisomere enthält.

5. Verfahren nach Ansprüchen 1 bis 4, dadurch gekennzeichnet, daß der rohe Nitrobenzaldehyd in trockner oder wasserfeuchter Form mit einem Wassergehalt von 0 -1000 Gew.-%, bevorzugt 1 - 800 Gew.-%, besonders bevorzugt 5 - 600 Gew.-%, bezogen auf die Gewichtsmenge trockenen Nitrobenzaldehyd, eingesetzt wird.

6. Verfahren nach Ansprüchen 1 bis 5, dadurch gekennzeichnet, daß man den rohen Nitrobenzaldehyd mit 150 - 3000 Gew.-% Wasser, bezogen auf das Trockengewicht des rohen Nitrobenzaldehyds, behandelt.

7. Verfahren nach Ansprüchen 1 bis 6, dadurch gekennzeichnet, daß zur Behandlung ein pH-Wert von 5 - 13 eingestellt wird.

8. Verfahren nach Ansprüchen 1 bis 7, dadurch gekennzeichnet, daß man zur Behandlung bei einer Temperatur von 40 - 120°C arbeitet.

9. Verfahren nach Ansprüchen 1 bis 8, dadurch gekennzeichnet, daß die Dosierung der Komponenten bei 10 - 140°C, bevorzugt 15 - 120°C, erfolgt.

10. Verfahren nach Ansprüchen 1 bis 9, dadurch gekennzeichnet, daß man den rohen Nitrobenzaldehyd mit 2 - 20 Gew.-% Emulgator, bezogen auf das Trockengewicht des rohen Nitrobenzaldehyds, behandelt.

**Claims**

1. Process for the purification of crude nitrobenzaldehyde which is contaminated by its positional isomers, characterised in that the crude nitrobenzaldehyde is treated with 100 - 10,000% by weight of water and 1 - 30% by weight of emulsifier, all relative to the dry weight of the crude nitrobenzaldehyde, at a temperature of 20 - 140°C and a pH of 3 - 14, so as to produce a two-phase system having a content of undissolved nitrobenzaldehyde.

2. Process according to Claim 1, characterised in that the crude nitrobenzaldehyde contains 0.1 - 15% by weight of undesired positional isomers.

3. Process according to Claims 1 and 2, characterised in that the crude nitrobenzaldehyde contains 0.1 - 10% by weight of undesired positional isomers.

4. Process according to Claims 1 to 3, characterised in that the crude nitrobenzaldehyde contains 0.1 - 8% by weight of undesired positional isomers.

5. Process according to Claims 1 to 4, characterised in that the crude nitrobenzaldehyde is employed in dry or water-moist form having a water content of 0 - 1,000% by weight, preferably 1 - 800% by weight, particularly preferably 5 - 600% by weight, relative to the weight amount of dry nitrobenzaldehyde.

6. Process according to Claims 1 to 5, characterised in that the crude nitrobenzaldehyde is treated with 150 - 3,000% by weight of water, relative to the dry weight of the crude nitrobenzaldehyde.

7. Process according to Claims 1 to 6, characterised in that the pH is adjusted to 5 - 13 for the treatment.

8. Process according to Claims 1 to 7, characterised in that a temperature of 40 - 120°C is used for the treatment.

9. Process according to Claims 1 to 8, characterised in that the metering of the components is carried out at 10 - 140°C, preferably 15 - 120°C.

**10.** Process according to Claims 1 to 9, characterised in that the crude nitrobenzaldehyde is treated with 2 - 20% by weight of emulsifier, relative to the dry weight of the crude nitrobenzaldehyde.

**Revendications**

**1.** Procédé de purification de nitrobenzaldéhyde brut, souillé par ses isomères de position, caractérisé en ce qu'on traite le nitrobenzaldéhyde brut avec 100 à 10 000% en poids d'eau et 1 à 30% en poids d'émulsionnant, rapportés au poids à sec de nitrobenzaldéhyde brut, à une température comprise entre 20 et 140°C et à un pH compris entre 3 et 14 de telle sorte qu'on obtient un système à deux phases comportant une certaine proportion de nitrobenzaldéhyde non dissous.

**2.** Procédé selon la revendication 1, caractérisé en ce que le nitrobenzaldéhyde brut contient de 0,1 à 15% en poids d'isomères de position indésirables.

**3.** Procédé selon les revendications 1 et 2, caractérisé en ce que le nitrobenzaldéhyde brut contient de 0,1 à 10% en poids d'isomères de position indésirables.

**4.** Procédé selon les revendications 1 à 3, caractérisé en ce que le nitrobenzaldéhyde brut contient de 0,1 à 8% en poids d'isomère de position indésirables.

**5.** Procédé selon les revendications 1 à 4, caractérisé en ce que le nitrobenzaldéhyde brut sous forme sèche ou humide d'eau est utilisé avec une teneur en eau comprise entre 0 et 1 000% en poids, de préférence entre 1 et 800% en poids, mieux encore entre 5 et 600% en poids, par rapport à la quantité en poids de nitrobenzaldéhyde sec.

**6.** Procédé selon les revendications 1 à 5, caractérisé en ce qu'on traite le nitrobenzaldéhyde brut avec 150 à 3 000% en poids d'eau, par rapport au poids à sec de nitrobenzaldéhyde brut.

**7.** Procédé selon les revendications 1 à 6, caractérisé en ce qu'on ajuste le pH entre 5 et 13 pour le traitement.

**8.** Procédé selon les revendications 1 à 7, caractérisé en ce qu'on travaille pour le traitement à une température comprise entre 40 et 120°C.

**9.** Procédé selon les revendications 1 à 8, caractérisé en ce qu'on ajoute les composants à une température comprise entre 10 et 140°C, de préférence entre 15 et 120°C.

**10.** Procédé selon les revendications 1 à 9, caractérisé en ce qu'on traite le nitrobenzaldéhyde brut avec 2 à 20% en poids d'émulsionnant, par rapport au poids à sec de nitrobenzaldéhyde brut.